# EUROPÄISCHE PATENTSCHRIFT

(11) **EP 1 004 286 B1**
(45) Veröffentlichungstag und Bekanntmachung des Hinweises auf die Patenterteilung: **27.10.2004**
(21) Anmeldenummer: 99122159.9
(22) Anmeldetag: 05.11.1999
(51) Int. Cl.: A61H 33/06, A61H 33/10

(54) **Vorrichtung zur Wärme- und/oder Dampfanwendung**
Apparatus for application of heat and/or steam
Appareil pour l'application de la chaleur et/ou la vapeur

(30) Priorität: 27.11.1998 DE 19854901
(43) Veröffentlichungstag der Anmeldung: 31.05.2000
(73) Patentinhaber: Franz, Josef Dr., 39031 Bruneck (IT)
(72) Erfinder: Franz, Josef Dr., 39031 Bruneck (IT)
(74) Vertreter: Niederkofler, Oswald A., Dipl.-Phys.

(56) Entgegenhaltungen:
- EP-A- 0 144 571
- DE-A- 2 202 014
- DE-A- 3 725 318
- FR-A- 1 413 510
- FR-A- 2 673 837
- US-A- 1 797 916
- US-A- 2 640 201

## Beschreibung

Die vorliegende Erfindung betrifft eine Vorrichtung zur Wärme- und/oder Dampfanwendung und deren Verwendung insbesondere zur Verabreichung von Heu-, Moor-, Schlick-, Algen-, Heilerde- oder Lehmbäder und/oder ätherischen Ölen.

Solche Wärmeanwendungen werden seit jeher zur Heil- und Therapiezwecken eingesetzt. Ein Beispiel hierfür sind die bei Kurbehandlungen häufig verwendeten Moorbäder, bei welchen der Anwender in einer erwärmten Moormasse eingetaucht ist und eine bestimmte Zeit dort verbleibt. In jüngster Zeit werden Wärme- und/oder Dampfanwendungen aber immer beliebter nicht nur in Verbindung mit Kurbehandlungen zu Heilzwecken, sondern auch zur Förderung des allgemeinen Wohlbefindens (sog. Wellness-Anwendung) oder unter kosmetischen Gesichtspunkten, und zwar auch für eine Anwendung zu Hause, in Hotels, in öffentlichen Badeanstalten, etc.. Für solche Zwecke sind die bisher bekannten Vorrichtungen zur Wärmeanwendung, wie etwa die genannten Moorbäder, weniger geeignet: sie sind teuer in der Anschaffung, weil sie einen relativ komplizierten Aufbau hinsichtlich der Erwärmung, Umwälzung und Zu- bzw. Ableitung des Wärmeträgermediums Moor benötigen. Auch in der Bedienung und Anwendung sind bekannte Wärmeanwendungsvorrichtungen umständlich und erfordern eine gleichermaßen umständliche Säuberung der gesamten Vorrichtung nach jeder Anwendung, um vorgeschriebenen hygienischen Standards gerecht zu werden.

Ein Beispiel für eine derartige Vorrichtung zur Wärmeanwendung ist aus der EP 0 144 571 A1 bekannt. Dort ist eine Wannenvorrichtung zum Anlegen von Wärmepackungen, insbesondere Schlammpackungen beschrieben. Die Wanneneinrichtung weist eine flüssigkeitsdichte Auflagefolie auf, welche über eine komplizierte Hebe- und Senkvorrichtung so verstellbar ist, daß eine darauf liegende Person zusammen mit den angelegten Wärmepackungen in die beispielsweise mit Moor aufgefüllte Wanne eintauchbar ist. Bereits dieser Aufbau bedingt eine aufwendige Bedienung der Vorrichtung, so daß eine Wärmebehandlung nur unter intensiver Betreuung und Aufsicht möglich ist. Ferner kommt hinzu, daß die fest montierte Auflagefolie, die insbesondere an den Badenden auf Grund der dort erforderlichen Übergröße Falten schlägt, nur umständlich zu reinigen ist und im Laufe der Zeit durch eine neue saubere Folie ersetzt werden muß.

Für die aus der EP 0 098 390 A1 bekannte Badevorrichtung gelten diese Ausführungen analog. Zwar verzichtet die dortige Vorrichtung z.B. für Moorvollbäder auf die aufwendige Senk- und Hebeeinrichtung. Trotzdem weist auch sie eine fest montierte flüssigkeitsdichte Trennfolie auf, auf welcher die Person liegt. Auch diese Trennfolie muß gegenüber der Wannenfläche zwangsläufig eine gewisse Übergröße aufweisen, damit die darauf liegende Person in die in die Wanne eingefüllte Moormasse eingetaucht werden kann. Eine geringe Moormenge wird auch in den Folienoberraum gefüllt; von diesem Moor muß die Trennfolie nach jeder Behandlung gereinigt werden, was sich auf Grund der Übergröße der Trennfolie und der damit einhergehenden Faltenbildung als besonders umständlich erweist.

Ferner ist aus der DE 22 02 04 C2 eine Vorrichtung zum Feinverteilen von unter anderem Luft, Dampf und Badezusätzen in einer Badewanne bekannt, bei der der Dampf durch Bohrungen in einem Rost in das Badewasser eingeblasen wird. Dabei sollen feinverteilte Luft und gegebenenfalls Badezusätze in der Nähe des Körpers des Badenden dem Badewasser in fein verteilter Form zugesetzt werden. Eine Dampfanwendung würde jedoch zu einer Verletzung des Badenden führen.

US-A-2 640 201 zeigt eine Badewanne mit Gitterauflage wobei Dampf durch Düsen am Boden der Wanne in kaltes Wasser zugeführt wird.

Schließlich offenbart die DE 37 25 318 C2 ein Verfahren zur Vorbereitung einer zum Beispiel Heubadeanwendung sowie einer Vorrichtung zur Herstellung eines entsprechenden Heubades. Dabei wird das vorbehandelte Heu auf einer dampfdurchlässigen Liegefläche oberhalb des Badewassers angeordnet. Der Badevorrichtung wird jedoch kein Wasserdampf zu einer Dampf- oder Wärmebehandlung zugeführt.

Die vorliegende Erfindung zielt darauf ab, eine Vorrichtung zur Wärme- und/oder Dampfanwendung zur Verfügung zu stellen, welche einfacher aufgebaut und vielseitiger einsetzbar ist als bekannte Vorrichtungen.

Die Erfindung erreicht dieses Ziel durch die Gegenstände der Ansprüche . Vorteilhafte bevorzugte Ausgestaltungen der Erfindung sind in den abhängigen Ansprüchen beschrieben.

Danach schafft die Erfindung eine Vorrichtung zur Wärmeund/oder Dampfanwendung an einer liegenden Person, welche unter Verwendung von Wasserdampf, der vorzugsweise von einer externen Dampfquelle zugeführt wird, die Erwärmung der Person sowie deren Umgebung auf unterschiedliche Weise ermöglicht: zum einen wird Wärmeenergie unmittelbar in Form von Wasserdampf zugeführt, gegebenenfalls unter Zusatz von Substraten mit entspannender, beruhigender, kosmetischer und/oder heilender Wirkung (sog. Dampfanwendung); und zum anderen wird Wärmeenergie durch Erhitzen von Wasser mittels Wasserdampf an die Umgebung und damit auch an die Person abgegeben (sog. Wärmeanwendung). Beide Erwärmungsvarianten, d.h. die Wärmeanwendung durch in der Wanne erhitztes Wasser und die Dampfanwendung durch unmittelbare Dampfzufuhr sind unabhängig voneinander, vorteilhaft aber auch in Kombination miteinander einsetzbar. Außerdem ist die Wärmeanwendung durch dampfunterstützes Erhitzten des Wassers am Wannenboden nicht auf eine reine Wärmeanwendung beschränkt, sondern geht selbstverständlich in eine Dampfanwendung über, wenn das Wasser bis zur Verdampfungstemperatur aufgeheizt wird.

Die erfindungsgemäße Vorrichtung weist hierfür auf: eine Wanne, welche mit kaltem und/oder heißem Wasser befüllbar ist; eine gas- bzw. dampfdurchlässige Auflageeinrichtung zur Aufnahme einer Person in liegender Position in einem Abstand vom Wannenboden; und eine Einrichtung zum Erhitzen von in die Wanne eingefülltem Wasser mittels Wasserdampf im Bereich des Wannenbodens und/oder eine Einrichtung zur Erzeugung oder Zufuhr von Wasserdampf im Bereich des Wannenbodens und/oder an wenigstens einer Wanneninnenseite.

Es ist ein Vorteil der Erfindung, daß an der Person in einer gewohnten und vertrauten Umgebung, nämlich in einer Art Badewanne mit einer herkömmlichen Einstiegshöhe sowie einem angenehmen Liegegefühl, wahlweise eine Wärmeanwendung durch Erhitzen des eingefüllten Wassers oder eine Dampfanwendung durch unmittelbare Dampfzufuhr oder die Kombination von beiden Anwendungen durchgeführt werden kann. Ferner kann die Erwärmung des Wasser auch nur genutzt werden, um die erfindungsgemäße Vorrichtung als solche aufzuheizen. Die auf der Auflageeinrichtung positionierte Person wird vorteilhaft ganzkörperlich oder wahlweise nur lokal, wie nachstehend noch näher erläutert wird, mit Wasserdampf umströmt.

Bei einer bevorzugten erfindungsgemäßen Vorrichtung zur Wärme- und/oder Dampfanwendung ist ein ausgewähltes Substrat direkt am Körper der Person und/oder auf der dampfdurchlässigen Auflageeinrichtung angeordnet, auf welchem die Person aufliegt. Dieses Substrat kann z.B. eine Schicht aus Heu sein. Die wohltuende und regenerierende Wirkung von Heu wurde zu früheren Zeiten bereits von den Tiroler Bergbauern erkannt und angewendet. Zur Regeneration hat man sich in Heu der ersten Heuernte, so bald dieses seinen Gärungsprozeß beginnt, hineingelegt und damit abgedeckt. Neben Heu sind auch andere beruhigende, entspannende, heilende und/oder kosmetisch wirkende Substratauflagen möglich, z.B. Schichten oder Packungen aus Moor, Heilerde, Heilschlamm, Algen oder Heilkräutern. Bei der erfindungsgemäßen Anwendung wird das Substrat direkt durch Wasserdampf und/oder indirekt durch die vom erhitzten Wasser angegebenen Wärmeenergie von unten erwärmt und gibt seine Wärme an die aufgelegte Person ab. Um die Wärme besser zu speichern, wird die Person bevorzugt mit einer weiteren Substratschicht und ggf. einer Abdeckung abgedeckt. Die erfindungsgemäße Wärmeund/oder Dampfanwendung in Verbindung mit einem ausgewählten Substrat hat den Vorteil, daß das Substrat als Wärmespeicher genutzt wird, zusätzlich aber auch sich im Substrat befindliche Heil- oder Regenerationsstoffe im Wasserdampf lösen und von der Person durch die Atemwege oder die Haut aufgenommen werden können.

Für die erfindungsgemäße Wärmeanwendung und ggf. auch Dampfanwendung sind bei einer bevorzugten Variante mehrere Rohrleitungen am Wannenboden offen verlaufend oder im Wannenboden integriert angeordnet, welchen von einer Dampfquelle Wasserdampf zugeführt wird, um das in die Wanne eingelassene Wasser zu erhitzen, vorzugsweise auch bis zum Verdampfen des Wassers. Bei der Inbetriebnahme der erfindungsgemäßen Vorrichtung wird beispielsweise Wasser in die Wanne bis zu einem relativ niedrigen Pegel eingelassen. Die dampfdurchströmten Rohrleitungen am oder im Wannenboden heizen das Wasserbad sodann auf, um die gesamte Vorrichtung zu erwärmen und für die Wärme- und ggf. Dampfanwendung vorzubereiten. Für die eigentliche Wärme- und ggf. Dampfanwendung wird vorzugsweise Wasser auf einen höheren Pegel in die Wanne nachgefüllt und durch die am/im Wannenboden geführten dampfbeheizten Rohrleitungen erhitzt, so daß die an das Wanneninnere abgegebene Wärmeenergie zur Erwärmung der Person bzw. des Substrats mit der Person führt (Wärmeanwendung). Wird das Wasser stärker bis zur Verdampfung erhitzt, so steigt der entstehende Wasserdampf auf und erwärmt auf diese Weise die Person bzw. das Substrat und die Person um so stärker (erweiterte Wärmeanwendung bzw. Dampfanwendung). Bevorzugt verfügt die Wanne über einen geregelten Wasserzulauf, derart, daß der eingefüllte Wasserpegel auf einem konstanten Wert gehalten wird, so daß eine kontinuierliche Wärme- bzw. Dampfanwendung möglich ist. Alternativ dazu wird bevorzugt das Wasserbad einmal aufgefüllt und der Wasserstand so bemessen, daß die Zeit für die vollständige Verdampfung des Wassers im wesentlichen einer Behandlungsdauer entspricht.

Ferner ist bevorzugt wenigstens ein Wasserzulauf, der ein steuerbares Ventil enthält, sowie ein Wasserpegelsensor im Bodenbereich der Wanne vorgesehen. Auf diese Weise kann der auf- und nachgefüllte Wasserpegel gezielt bemessen und der Auf- und Nachfüllvorgang automatisiert werden. Außerdem ist bevorzugt auch ein Temperatursensor im Bodenbereich der Wanne zum Messen der Wassertemperatur vorgesehen, und die Dampfquelle in Reaktion auf die vom Temperatursensor empfangenen Signale bezüglich des abgegebenen Dampfdruckes mit Rücksicht auf die gewünschte Wassertemperatur steuerbar ausgebildet.

Bei einer bevorzugten Variante in Hinblick auf die Dampfanwendung der erfindungsgemäßen Vorrichtung sind wenigstens an einer Innenseite der Wanne, vorzugsweise an beiden langen Innenseiten der Wanne, eine oder mehrere Dampfdüsen angeordnet, welche von einer Dampfquelle mit Wasserdampf versorgt werden und diesen sprühkegelartig in den Wanneninnenraum abgeben. Vorzugsweise sind diese Dampfdüsen bezüglich Ihres Abstrahlwinkels verstellbar ausgebildet, z.B. als Düse mit gelenkig gelagerten Düsenkopf, um eine gerichtete und direktere Dampfbeaufschlagung der Person bzw. des Substrats mit Person zu ermöglichen. Bei einer weiteren bevorzugten Ausführungsform weist/weisen jede Dampfzufuhrleitung, welche einer bestimmten Düse zugeordnet ist, ein eigenes steuerbares Ventil oder mehrere zu Gruppen zusammengefaßte Dampfzufuhrleitungen ein gemeinsames Steuerventil auf, so daß einzelne ausgewählte Düsen oder ausgewählte Gruppen von Düsen zur lokalen Dampfanwendung gespeist werden können.

Um die lokale oder ganzkörperliche Dampfanwendung überwachen zu können, ist bevorzugt an Temperatursensor im Bereich der Auflageeinrichtung vorgesehen und die Dampfquelle in Reaktion auf die vom Temperatursensor empfangenen Signale bezüglich des abgegebenen Dampfdruckes mit Rücksicht auf die gewünschte Dampfabgabe der Dampfdüsen steuerbar. Wird bei der Dampfanwendung beispielsweise eine vorgegebene Grenztemperatur überschritten, so kann der Dampfdruck der Dampfquelle nach einem vorgegebenen Programm automatisch zurück geregelt werden, so daß auch die durch die Düsen abgegebene Dampfmenge entsprechend abnimmt, bis die gewünschte Behandlungstemperatur wieder erreicht ist.

Die oben erwähnte Dampfquelle zur Aufbereitung des Wasserdampfes kann in der erfindungsgemäßen Vorrichtung integriert sein. Vorzugsweise ist aber die Dampfquelle extern von der erfindungsgemäßen Vorrichtung angeordnet und bezüglich des abgegebenen Dampfdruckes steuer- bzw. regelbar, um z.B. den Rohrleitungen und/oder den Dampfdüsen Wasserdampf bei einem vorgegebenen Dampfdruck zuzuführen. Zur individuellen Anpassung der erfindungsgemäßen Wärmeund/oder Dampfanwendung ist zusätzlich vorzugsweise eine Steuerungseinrichtung vorgesehen, die in Reaktion auf die von den Wasserpegel- und/oder Temperatursensoren empfangenen Signale die von den dampfbeheizten Rohrleitungen abgegebene Wärmeenergie bzw. die von den Dampfdüsen abgegebene Dampfmenge so steuert, daß der Wärme- und/oder Dampfanwendungsverlauf der erfindungsgemäßen Vorrichtung entweder einem vorgegebenen Programm entspricht oder durch manuell betätigbare Steuerschalter bestimmbar ist. Aus Gründen der Sicherheit verfügt diese Steuerungseinrichtung außerdem über eine Niedrigvolt-Stromversorgungsquelle für die Sensoren und/oder steuerbaren Ventile der erfindungsgemäßen Vorrichtung, welche von dem wasserführenden Komponenten der Vorrichtung in geeigneter Weise isoliert sind.

Die Wanne der erfindungsgemäßen Vorrichtung zur Wärmeund/oder Dampfanwendung ist bevorzugt mit einem sich zum Wannenboden hin verjüngenden Querschnittsprofil ausgebildet. Dies hat den Vorteil, daß zum Auffüllen der Wanne bis zu einem bestimmen Wasserstand deutlich weniger Wasser verbraucht wird, als bei bekannten Wannen, welche z.B. die Form einer herkömmlichen Badewanne haben. Die erfindungsgemäße Wannenform ist damit vergleichsweise energiesparend.

Bei einer weiteren bevorzugten Variante der erfindungsgemäßen Vorrichtung ist in einer Seitenwand der Wanne, z.B. unmittelbar am oberen Wannenrand oder vom oberen Wannenrand aus bis zum Wannenboden verlaufend, ein Einlauf vorgesehen, über welchen ausgewählte Extrakte mit beruhigender, entspannender, kosmetischer und/oder heilender Wirkung dem Wasserbad zugesetzt werden können.

Ferner ist die Auflageeinrichtung bevorzugt als netzartiges flexibles Gitter, vorzugsweise aus Kunststoff, ausgebildet, welches an den Wanneninnenseiten abnehmbar befestigt ist. Dieses Auflagegitter hat den Charakter einer Hängematte und ist daher für eine bequeme Aufnahme eines Probanden in liegender Position geeignet; sie ist außerdem ist leicht vom Gewicht und läßt sich zur z.B. Reinigung aus der erfindungsgemäßen Vorrichtung ohne Schwierigkeiten herausnehmen. Bevorzugt weist hierfür die Auflageeinrichtung an beiden Längsseiten jeweils durchgehende Befestigungsstäbe auf, die in entsprechende Befestigungshaken an den länglichen Innenseiten der Wanne zur Fixierung der Auflagevorrichtung eingehängt werden können. Zur Erhöhung des Sicherheitsstandards der erfindungsgemäßen Vorrichtung ist außerdem bevorzugt ein gelochtes Sicherungsgitter, vorzugsweise aus Stahl oder Kunststoff, in einem Abstand unterhalb der Auflageeinrichtung vorgesehen, welches ebenfalls vorteilhaft abnehmbar an den Innenseite der Wanne angebracht ist.

Aus der nachfolgenden Beschreibung eines bevorzugten Ausführungsbeispiels ergeben sich weitere Vorteile und Merkmale der Erfindung. In dieser Beschreibung wird auf die einzige schematische Figur Bezug genommen, in welcher eine stirnseitige Querschnittsdarstellung einer erfindungsgemäßen Vorrichtung zur Wärme- und/oder Dampfanwendung gezeigt ist.

Die erfindungsgemäße Vorrichtung zur Wärme- und/oder Dampfanwendung an einer Person 2 umfaßt eine Edelstahlwanne 4, welche im wesentlichen die Form eines nach oben hin offenen Kastens hat und der Länge und Breite nach so bemessen ist, daß mindestens eine erwachsene Person mit ausreichendem Abstand zum Wannenrand in liegender Position aufgenommen werden kann. Aus Gründen der Energie- und Wasserersparnis ist die Wanne 4 mit einem sich zum Wannenboden hin verjüngenden Querschnittsprofil ausgebildet. Hierbei hat die Wanne 4 in der oberen Hälfte einen im wesentlichen rechteckigen Querschnitt, der sich mit einem ersten steileren und einem anschließenden flacheren Abschnitt zum Wannenboden hin verjüngt. Die Wannenaußenseite ist mit einer hier nicht gezeigten Isolierschicht umgeben; ferner ist ein äußeres Gestell mit Verkleidung vorgesehen, welches bevorzugt auf Rollen geführt ist.

In einem geringen Abstand zum Boden der Wanne 4 sind mehrere parallele, in Längsrichtung verlaufende offene Rohrleitungen 6, z. B. aus einer Edelstahllegierung, in einem Abstand zueinander verlegt. Die Rohrleitungen 6 sind über Anschlußstücke an jeweils beiden Rohenden an eine gemeinsame Umlaufleitung 8 angeschlossen, welche ihrerseits über eine Dampfzufuhrleitung 12 mit einem externen Dampfgenerator 10 verbunden ist. Diese versorgt die Rohrleitungen 6 mit Wasserdampf eines bestimmten einstellbaren Druckes. Dabei zirkuliert der Wasserdampf durch die Umlaufleitung 8 und die daran angeschlossenen Rohrleitungen 6, welche erhitzt werden und die Wärme an das umliegende Wasser abgeben. Nach Abgabe der Wärmeenergie wird das in den Rohrleitungen 6 übrig bleibende Wasser in einem (nicht gezeigten) mit der Umlaufleitung 8 verbundenen Auffangbehälter gesammelt und abgeführt.

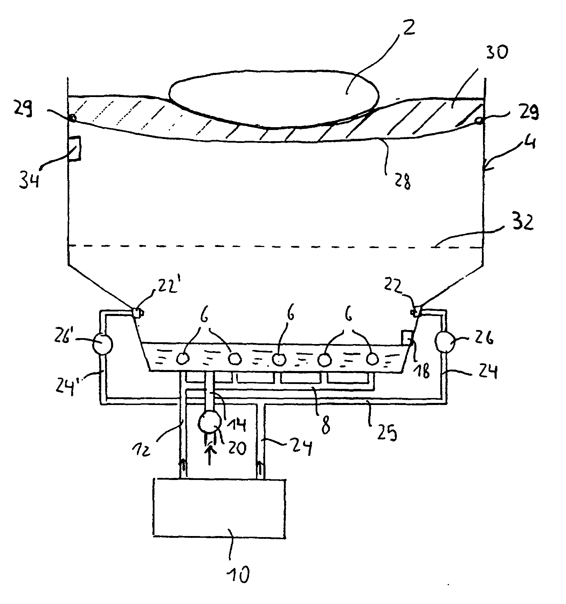

Über einen Wasserzulauf 14 kann kaltes Wasser in die Wanne 4 eingelassen werden. Ferner ist bevorzugt ein weiterer - nicht gezeigter - Warmwasserzulauf vorgesehen. Wie in der Figur schematisch dargestellt ist, umspült das eingelassene Wasser die dampfdurchströmten Rohrleitungen 6 am Boden der Wanne 4, so daß das Wasser durch die Rohrleitungen 6 erhitzt wird. Ferner ist an einer Innenseite der Wanne 4 in einem relativ geringen Abstand vom Wannenboden ein Wasserpegelsensor 18 angebracht, um den Wassereinfüllvorgang bei Erreichen des Wasserstandes auf Höhe des Pegelsensors 18 automatisch zu beenden. Hierfür erhält eine nicht näher dargestellte Steuerungseinrichtung ein Signal des Pegelsensors 18 und steuert daraufhin ein steuerbares Ventil 20, welches in der Wasserzulaufleitung 14 vorgesehen ist, so daß der Wasserzulauf 14 bei Erreichen des vorgegebenen Wasserpegels gesperrt wird.

Außerdem sind an den beiden langen Innenseiten der Wanne 4 im Bereich des Übergangs vom flachen zum steilen konischen Wannenabschnitt mehrere schwenkbare Dampfdüsen 22, 22' vorgesehen, wobei die Düsen 22, 22' über individuelle Versorgungsanschlüsse 24, 24' an einer Umlaufleitung 25 angeschlossen sind, die ihrerseits über eine einzige - von der Zufuhrleitung 12 gesonderte - Dampfzufuhrleitung 26 mit der Dampfquelle 10 verbunden ist. Darüber hinaus sind in den Anschlußleitungen 24, 24', welche zu den einzelnen Düsen 22, 22' führen, jeweils steuerbare Ventile 26, 26' eingebaut, welche im Zusammenspiel mit der oben erwähnten Steuerungseinrichtung dafür sorgen, daß Wasserdampf aus der Dampfquelle 10 je nach Wunsch nur über eine oder mehrere ausgewählte Dampfdüsen 22, 22' zugeführt wird.

Zur Lagerung der Person 2 ist ein netzartiges Gitter 28 nach Art einer Hängematte vorgesehen, welches etwas unterhalb des Wannenoberrandes an den Innenseiten der Wanne 4 abnehmbar befestigt ist. Hierfür sind an den beiden Längsseiten des netzartigen Gitters 28 jeweils durchgehende Befestigungsstäbe 29 eingelassen. An den beiden langen Innenseiten der Wanne 4 sind mehrere zugehörige Befestigungshaken (nicht gezeigt) auf einer Höhe etwas unterhalb des Wannenoberrandes vorgesehen, an welchen die Befestigungsstangen 29 samt Gitter 28 ein- und ausgehängt werden können. Auf diese Weise ist das netzartige Gitter 28 für z.B. Reinigungszwecke nach einer durchgeführten Wärme- und/oder Dampfanwendung mit einem einzigen Handgriff aus der Wanne 4 entfernbar. Aufgrund der netzartigen Struktur ist das Gitter 28 dampfdurchlässig. Auf dem Gitter 28 ist das für eine spezifische Wärme- und/oder Dampfanwendung verwendete Substrat 30 aufgebracht, z.B. eine Schicht aus Heu. Auf diesem Substrat 30 liegt dann die Person 2 und ist vorzugsweise mit einer weiteren Schicht aus dem gleichen Substrat 30 oder einem anderen Substrat mit einer zusätzlichen Abdeckung abgedeckt (in der Figur nicht gezeigt).

Zur Sicherung der Person 2 vor einem ungewollten Kontakt mit dem darunter liegenden Dampf- und Wärmequellen ist in einem Abstand von dem Auflagegitter 28 ferner ein gelochtes Metallgitter 32 angeordnet, welches an der Wanneninnenseite ebenfalls abnehmbar angebracht ist. Hierfür ist der äußere Rand des Metallgitters 32 nach unten gewölbt und kann hierdurch an einer an der Wanneninnseite verlaufenden Schiene ein- und wieder ausgehängt werden.

Darüber hinaus ist unmittelbar unterhalb des Auflagegitters 28 an der Wanneninnenseite ein Temperatursensor 34 zur Temperaturüberwachung der Dampf- und/oder Wärmeanwendung vorgesehen. Die nicht gezeigte Steuerungseinrichtung empfängt Signale des Temperatursensors 34. In Reaktion darauf ist der Dampfdruck der Dampfquelle 10 entweder manuell oder automatisch einstellbar - und damit auch die über die Düsen 22, 22' und/oder an die Rohrleitungen 6 abgegebene Dampfmenge. Außerdem werden die steuerbaren Ventile an den Zufuhrleitungen 24 der Düsen 22, 22' in Reaktion auf die vom Temperatursensor 34 gemessenen Temperaturen in unmittelbarer Nähe der Person 2 von der Steuerungseinrichtung in gewünschter Weise manuell gesteuert oder automatisch geregelt.

Mit der zuvor erwähnten Schalt- und Steuerungseinrichtung kann beispielsweise der nachfolgend näher beschriebene Bedienungsablauf zur Wärme- und/oder Dampfanwendung ausgeführt werden.

Zu Beginn der Inbetriebnahme wird die Steuerungseinrichtung auf einen "Vorwärmmodus" eingestellt. In diesem Betriebsmodus wird Wasser über den Zulauf 14 in die Wanne 4 eingelassen, bis der Wasserpegelsensor 18 anschlägt und das Steuerungsventil 20 im Wasserzulauf 14 automatisch gesperrt wird. Gleichzeitig wird durch den Dampfgenerator 10 Wasserdampf mit relativ geringem Druck erzeugt. Der Wasserdampf strömt durch die Rohrleitungen 6 am Wannenboden und erhitzt hierdurch das die Rohrleitungen 6 umströmende Wasser bis zu einer Temperatur von ca. 45°C bis 60°C. Das erhitzte Wasser gibt seine Wärmeenergie an die Umgebung ab, erwärmt dabei den Wanneninnenraum, die Edelstahlwanne 4 und gegebenenfalls das bereits auf dem Auflagegitter 28 befindliche Substrat 30. Der "Vorwärmmodus" dient vor allem der Vorbereitung der erfindungsgemäßen Vorrichtung für die anschließende Wärme- und/oder Dampfanwendung, so daß die Person 2 in einer behaglichen Atmosphäre in der Vorrichtung Platz nehmen kann. Wenn nun die Person 2 auf dem Substrat 30 liegt und gegebenenfalls mit einer weiteren Substratschicht abgedeckt ist, wird eine Dampfanwendung an der Person durchgeführt. Hierfür wird die Steuerungseinrichtung auf den "Dampfanwendungsmodus" gestellt, so daß die Dampfdüsen 22, 22' über die Leitung 24 mit Wasserdampf auf relativ geringem Druck und mit geringer Feuchtigkeit versorgt werden. Zur lokalen Dampfanwendung können auch nur ausgewählte Düsen durch die Steuerungseinrichtung angesprochen werden oder nach einem vorgegebenen Steuerungsprogramm unterschiedliche Düsen nacheinander mit Wasserdampf beaufschlagt werden.

Es sei hierbei aber nochmals betont, daß auch nur eine reine Wärmeanwendung, ohne Dampfanwendung, durch Erhitzen des Wasserbades durch die temperatursteuerbaren Dampfrohrleitungen 6 am Boden der Wanne 4 oder eine reine Dampfanwendung durch die Dampfzufuhr an den verstellbaren und individuell ansprechbaren und temperatursteuerbaren Dampfdüsen 22, 22' sowie jede beliebige Kombination dieser beiden Anwendungen möglich ist.

Zusammengefaßt ergeben sich danach bei der erfindungsgemäßen Vorrichtung zur Wärme- und/oder Dampfanwendung folgende Vorteile:
- eine optimale Erhitzung des Wärmeträgers Wasser in der Wanne durch die am Wannenboden geführten mit Wasserdampf erhitzten Rohre. Diese Erhitzung findet vorteilhaft völlig ohne Bedarf eines z.B. für eine Tauchsiedevorrichtung notwendigen Stromkreises statt;
- energiesparende Form der Wanne;
- ganzkörperliche oder lokal beschränkte Erwärmung einer Person durch regelbare Dampfdüsen oberhalb des Wärmeträgers Wasser;
- die Kombination beider Erwärmungsmöglichkeiten durch Dampf und Wasser in der Wanne;
- der Einsatz von Wasser und Dampf als Wärmeträger ist im Ergebnis optimal steuerbar und differenziert für kosmetische Anwendungen, sog. Wellness-Anwendungen, andere Anwendungen, etc., für Bäder mit verschiedenen Substraten oder unmittelbar am Körper der Person einsetzbar; und
- die bedienungsfreundliche und hygienische Ausführung der Wanne mit einfacher Einhängung der hängemattenartigen Auflageeinrichtung.

## Patentansprüche

1. Vorrichtung zur Wärme- und/oder Dampfanwendung, mit:
a) einer Wanne (4), welche mit Wasser befüllbar ist,
b) einer dampfdurchlässigen Auflageeinrichtung (28) zur Aufnahme einer Person in liegender Position in einem Abstand vom Wannenboden,
c) einer Einrichtung (22, 24, 25, 27) zur Zufuhr von Wasserdampf im Bereich des Wannenbodens und/oder an wenigstens einer Wanneninnenseite,
**dadurch gekennzeichnet, daß**
d) eine Einrichtung (6, 8, 12) zum Erhitzen von in die Wanne (4) eingefülltem Wasser mittels Wasserdampf im Bereich des Wannenbodens vorgesehen ist, wobei die beiden Einrichtungen (22, 24, 25, 27; 6, 8, 12) von derselben Dampfquelle (10) mit Wasserdampf gespeist sind.

2. Vorrichtung nach Anspruch 1, **dadurch gekennzeichnet, daß** direkt am Körper der Person und/oder auf der Auflageeinrichtung (28) ein ausgewähltes Substrat (30) angeordnet ist, auf welchem die Person (2) aufliegt.

3. Vorrichtung nach Anspruch 1 oder 2, **dadurch gekennzeichnet, daß** mehrere von Wasserdampf durchströmte Rohrleitungen (6) am Wannenboden offen verlaufen und vom in die Wanne gefülltem Wasser umspült werden, um das Wasser in der Wanne (4) zu erhitzen.

4. Vorrichtung nach Anspruch 3, **dadurch gekennzeichnet, daß** die Wanne (4) über einen geregelten Wasserzulauf (14) verfügt, derart, daß der Wasserpegel auf einem konstanten Wert gehalten wird.

5. Vorrichtung nach Anspruch 4, **dadurch gekennzeichnet, daß** im Wasserzulauf (14) ein steuerbares Ventil (20) und im Bodenbereich der Wanne (4) ein Wasserpegelsensor (18) und/oder ein Wassertemperatursensor vorgesehen sind.

6. Vorrichtung nach einem der vorstehenden Ansprüche, **dadurch gekennzeichnet, daß** wenigstens an einer Innenseite der Wanne (4) eine oder mehrere Dampfdüsen (22, 22') vorgesehen sind, welchen Wasserdampf zugeführt wird.

7. Vorrichtung nach einem der Ansprüche 3 bis 6, **gekennzeichnet durch** eine externe Dampfquelle (10), welche den Rohrleitungen (6) und/oder den Dampfdüsen (22, 22') Wasserdampf bei vorgegebenem Dampfdruck zugeführt.

8. Vorrichtung nach Anspruch 6 oder 7, **dadurch gekennzeichnet, daß** die Dampfdüsen (22, 22') bezüglich ihres Abstrahlwinkels verstellbar ausgebildet sind.

9. Vorrichtung nach einem der Ansprüche 6 bis 8, **dadurch gekennzeichnet, daß** jede Dampfzufuhrleitung (24, 24'), welche einer bestimmten Dampfdüse (22, 22') zugeordnet ist, ein steuerbares Ventil aufweist, oder mehrere zu Gruppen zusammengefaßte Dampfzufuhrleitungen ein gemeinsames Steuerventil aufweisen.

10. Vorrichtung nach einem der Ansprüche 7 bis 9, **dadurch gekennzeichnet, daß** ein Temperatursensor (34) im Bereich der Auflageeinrichtung (28) an der Wanneninnenseite vorgesehen ist, und die Dampfquelle (10) in Reaktion auf die vom Temperatursensor (34) empfangenen Signale bezüglich des abgegebenen Dampfdruckes mit Rücksicht auf die gewünschte Dampfabgabe der Dampfdüsen (22, 22') steuerbar ist.

11. Vorrichtung nach einem der Ansprüche 5 bis 10, **dadurch gekennzeichnet, daß** eine Steuerungseinrichtung vorgesehen ist, welche in Reaktion auf die von Wasserpegel- und/oder Temperatursensoren (18, 34) empfangenen Signale die von den Rohrleitungen (6) abgegebene Wärmeenergie bzw. die von den Dampfdüsen (22, 22') abgegebene Dampfmenge nach einem vorgegebenen Programm oder aufgrund manueller Eingaben steuert.

12. Vorrichtung nach einem der vorstehenden Ansprüche, **dadurch gekennzeichnet, daß** die Wanne (4) mit einem sich zum Wannenboden hin verjüngenden Querschnittsprofil ausgebildet ist.

13. Vorrichtung nach einem der vorstehenden Ansprüche, **dadurch gekennzeichnet, daß** am oberen Wannenrand oder in einer Seitenwand der Wanne (4) ein Einlauf vorgesehen ist, über welchen ausgewählte Extrakte mit beruhigender, entspannender, kosmetischer und/oder verschiedener Wirkung dem Wasserbad zusetzbar sind.

14. Vorrichtung nach einem der vorstehenden Ansprüche, **dadurch gekennzeichnet, daß** die Auflageeinrichtung (28) als netzartiges flexibles Gitter ausgebildet ist, welches an den Wanneninnenseiten abnehmbar befestigt ist.

15. Vorrichtung nach Anspruch 14, **dadurch gekennzeichnet, daß** das an den Längsseiten der Auflageeinrichtung (28) jeweils durchgehende Befestigungsstangen (29) vorgesehen sind, welche an Befestigungshaken an den Wanneninnenseiten einhängbar sind.

16. Vorrichtung nach einem der vorstehenden Ansprüche, **dadurch gekennzeichnet, daß** ein gelochtes Sicherungsgitter (32) in einem Abstand unterhalb der Auflageeinrichtung (28) vorgesehen ist, welches abnehmbar an den Innenseiten der Wanne (4) angebracht ist.

## Claims

1. An apparatus for the application of heat and/or steam comprising:
a) a tub (4) which can be filled with water,
b) a steam-permeable support (28) for resting a person in a lying position spaced away from the bottom of said tub,
c) a means (22, 24, 25, 27) for supplying steam in the region of said tub bottom and/or of at least one inner side of said tub,
**characterized in that**
d) a steaming means (6, 8, 12) is provided in the region of said tub bottom for heating water filled into the tub (4), both means (22, 24, 25, 27; 6, 8, 12) being fed with steam from the same steam source (10).

2. The apparatus as set forth in claim 1, **characterized in that** arranged directly contacting the body of said person and/or said support (28) is a selected substrate (30) on which said person (2) lies.

3. The apparatus as set forth in claim 1 or 2, **characterized in that** several pipes (6) with a throughflow of steam are routed exposed on the bottom of said tub and are surrounded by the flow of water filled into the tub for heating the water in said tub (4).

4. The apparatus as set forth in claim 3, **characterized in that** said tub (4) features a regulated water feed (14) such that said water level is maintained constant.

5. The apparatus as set forth in claim 4 **characterized in that** provided in said water feed (14) is a controllable valve (20) and in the bottom portion of said tub (4) a water level sensor (18) and/or a water temperature sensor is provided.

6. The apparatus as set forth in any of the preceding claims, **characterized in that** one or more steam nozzles (22, 22') are provided on at least one inner side of said tub (4) for the supply of steam.

7. The apparatus as set forth in any of the claims 3 to 6, **characterized by** an external steam source which supplies steam at a prescribed steam pressure to said pipes (6) and/or said steam nozzles (22, 22').

8. The apparatus as set forth in claim 6 or 7, **characterized in that** said steam nozzles (22, 22') are configured adjustable as regards their jetting angle.

9. The apparatus as set forth in any of the claims 6 to 8, **characterized in that** each steam feeder (24, 24') assigned a specific steam nozzle (22, 22') comprises a controllable valve, or several steam feeders grouped together comprise a common control valve.

10. The apparatus as set forth in any of the claims 7 to 9, **characterized in that** a temperatur sensor (34) is provided in the region of said support (28) at the inner side of said tub, and said steam source (10) is controllable in response to signals as regards said jetted steam pressure with regard to said desired steam jetting of said steam nozzles (22, 22') as received by the temperatur sensor (34).

11. The apparatus as set forth in any of the claims 5 to 10, **characterized in that** a controller is provided which in response to signals received from the water level and/or temperatur sensors controls the thermal energy given off by the pipes (6) or the amount of steam given off by the steam nozzles (22, 22') as programmed or manually entered.

12. The apparatus as set forth in any of the preceding claims, **characterized in that** said tub (4) is configured with a cross-sectional profile tapered towards said tub bottom.

13. The apparatus as set forth in any of the preceding claims, **characterized in that** provided at the upper rim or sidewall of said tub (4) is an infeed via which selected extracts having a soothing, relaxing, cosmetic and/or various other effects can be added to said water bath.

14. The apparatus as set forth in any of the preceding claims, **characterized in that** said support (28) is configured as a flexible mesh structure removably connected to the inner sides of said tub.

15. The apparatus as set forth in claim 14, **characterized in that** fastener rods are provided over the full length of said support (28) for attachment to hook connectors on the inner sides of said tub.

16. The apparatus as set forth in any of the preceding claims, **characterized in that** a perforated security mesh is provided removably mounted on the inner sides of said tub (4) spaced away below said support (28).

## Revendications

1. Dispositif pour l'application de la chaleur et/ou de la vapeur, comprenant:
a) une baignoire (4), remplissable d'eau,
b) un moyen de support (28) perméable à la vapeur pour recevoir une personne en position couchée à une distance du fond de la baignoire.
c) un moyen d'alimentation en vapeur d'eau (22, 24, 25, 27) au niveau du fond de la baignoire et/ou à au moins un des flancs intérieurs de la baignoire,
**caractérisé en ce que**
d) un moyen de chauffage (6, 8, 12) de l'eau remplie dans la baignoire (4) par vapeur d'eau est prévu au niveau du fond de la baignoire, ces deux moyens (22, 24, 25, 27; 6, 8, 12) étant alimentés par la même source (10) de vapeur d'eau.

2. Dispositif selon la revendication 1, **caractérisé en ce qu'**un substrat choisi (30), sur lequel la personne est étendue, est disposé directement sur le corps de la personne (2) et/ou sur le moyen de support (28).

3. Dispositif selon la revendication 1 ou 2, **caractérisé en ce que** plusieurs tuyaux (6) parcourus de vapeur d'eau s'étendent ouvertement dans le fond de la baignoire et sont baignés de tous côtés par l'eau remplie dans la baignoire afin de chauffer l'eau dans la baignoire (4).

4. Dispositif selon la revendication 3, **caractérisé en ce que** la baignoire (4) dispose d'une arrivée d'eau réglée (14), de sorte que le niveau d'eau soit maintenu à valeur constante.

5. Dispositif selon la revendication 4, **caractérisé en ce qu'**au niveau de l'arrivée d'eau une soupape contrôlable (20) et au niveau du fond de la baignoire (4) un détecteur de niveau d'eau (18) et/ou un détecteur de température d'eau sont prévus.

6. Dispositif selon l'une quelconque des revendications précédentes, **caractérisé en ce qu'**un ou plusieurs injecteurs (22, 22') de vapeur alimentés de vapeur d'eau sont prévus sur au moins l'un des flancs intérieurs de la baignoire (4).

7. Dispositif selon l'une quelconque des revendications 3 à 6, **caractérisé par** une source (10) externe de vapeur qui alimente les tuyaux (6) et/ou les injecteurs de vapeurs (22, 22') en vapeur d'eau à une pression de vapeur donnée.

8. Dispositif selon la revendication 6 ou 7, **caractérisé en ce que** des injecteurs (22, 22') sont conçus orientables suivant leur angle d'éjection.

9. Dispositif selon l'une quelconque des revendications 6 à 8, **caractérisé en ce que** chaque conduite d'alimentation (24, 24') en vapeur correspondant à un injecteur (22, 22') particulier, comprend une soupape contrôlable, ou que plusieurs conduites d'alimentation groupées comprennent une soupape contrôlable commune.

10. Dispositif selon l'une quelconque des revendications 7 à 9, **caractérisé en ce qu'**un détecteur de température (34) est prévu au niveau du moyen de support (28) sur le flanc intérieur de la baignoire, et que la source (10) de vapeur est contrôlable en réaction aux signaux reçus par le détecteur de température (34) par rapport à la pression délivrée en prenant en compte la quantité souhaitée de vapeur délivrée par les injecteurs (22, 22') de vapeur.

11. Dispositif selon l'une quelconque des revendications 5 à 10, **caractérisé en ce qu'**un système de contrôle est prévu, qui contrôle l'énergie calorifique émise par les tuyaux (6) ou bien la quantité de vapeur délivrée par les injecteurs (22, 22') de vapeurs selon un programme donné ou manuellement, en réaction aux signaux reçus par les détecteurs de température ou du niveau d'eau (18, 34).

12. Dispositif selon l'une quelconque des revendications précédentes, **caractérisé en ce que** la baignoire (4) est formée d'un profil en coupe se rétrécissant vers le fond de la baignoire.

13. Dispositif selon l'une quelconque des revendications précédentes, **caractérisé en ce qu'**au niveau supérieur du bord de la baignoire (4) ou sur l'un des flancs de la baignoire (4) une conduite est prévue, par laquelle on peut ajouter dans le bain des extraits choisis à effets apaisant, relaxant, cosmétique, et/ou autre.

14. Dispositif selon l'une quelconque des revendications précédentes **caractérisé en ce que** le moyen de support (28) est formé d'une grille flexible en forme de filet fixée de façon démontable sur les flancs intérieurs de la baignoire.

15. Dispositif selon la revendication 14, **caractérisé en ce que** sur chacun des longs côtés du moyen de support (28), des baguettes de fixation (29) ininterrompues sont prévues, qui sont suspensives à des fixations prévues sur les flancs intérieurs de la baignoire.

16. Dispositif selon l'une des quelconques revendications précédentes, **caractérisé en ce qu'**une grille de sécurité (32) trouée qui est placée de façon amovible aux flancs intérieurs de la baignoire(4), est prévue à une distance en dessous du moyen de support (28).
